# EUROPÄISCHE PATENTSCHRIFT

(11) **EP 3 175 789 B1**
(45) Veröffentlichungstag und Bekanntmachung des Hinweises auf die Patenterteilung: **26.12.2018**
(21) Anmeldenummer: 16197861.4
(22) Anmeldetag: 09.11.2016
(51) Int. Cl.: A61B 6/00

(54) **VERFAHREN ZUR BILDUNTERSTÜTZUNG EINES BEHANDLERS, RÖNTGENEINRICHTUNG UND COMPUTERPROGRAMM**
METHOD FOR PROVIDING IMAGE SUPPORT OF A TREATMENT PROVIDER, X-RAY DEVICE AND COMPUTER PROGRAM
PROCÉDÉ DE PRISE EN CHARGE D'IMAGE, ÉQUIPEMENT À RAYONS X ET PROGRAMME INFORMATIQUE

(30) Priorität: 04.12.2015 DE 102015224356
(43) Veröffentlichungstag der Anmeldung: 07.06.2017
(73) Patentinhaber: Siemens Healthcare GmbH, 91052 Erlangen (DE)
(72) Erfinder: Hoffmann, Thomas, 39108 Magdeburg (DE); Skalej, Martin, 39114 Magdeburg (DE)

(56) Entgegenhaltungen:
- WO-A1-2015/075047
- US-A1- 2013 116 551
- US-A1- 2015 213 600

## Beschreibung

Die Erfindung betrifft ein Verfahren zur Bildunterstützung eines Behandlers bei der interventionellen Behandlung eines Zielgebiets eines Patienten, insbesondere bei der minimalinvasiven Behandlung im Blutgefäßsystem des Patienten. Daneben betrifft die Erfindung eine Röntgeneinrichtung und ein Computerprogramm.

Minimalinvasive Eingriffe mit medizinischen Instrumenten, beispielsweise Kathetern, eröffnen neue und für den Patienten leichter verkraftbare Möglichkeiten zur Behandlung von Pathologien/Läsionen, beispielsweise Aneurysmen. Um die Nachverfolgung des medizinischen Instruments und/oder den Behandlungsfortschritt überwachen zu können, wurde bereits vorgeschlagen, den interventionellen, insbesondere minimalinvasiven, Eingriff unter Bildüberwachung durchzuführen. Hierzu wird meist die Fluoroskopie verwendet, das bedeutet, mit einer Röntgeneinrichtung aufgenommene Röntgenbilder, für die eine eher geringe Röntgendosis gewählt wird.

Ein weiteres wichtiges Hilfsmittel zur Bildunterstützung bei einem interventionellen Eingriff im Blutgefäßsystem des Patienten ist die Subtraktionsangiographie. Dabei wird dem Patienten ein Kontrastmittel in den Blutkreislauf verabreicht, welches im Röntgenbild deutlich sichtbar ist. Durch Subtraktion eines ohne Kontrastmittel aufgenommenen Maskenbildes von einem Röntgenbild mit wenigstens teilweise kontrastmittelgefüllten Gefäßen (Füllungsbild) lässt sich ein Subtraktionsangiographiebild erhalten, welches lediglich die mit Kontrastmittel gefüllten Bereiche, mithin die Blutgefäße, zeigt. Nimmt man Projektionsbilder unter verschiedenen Projektionsrichtungen auf und nutzt man Verfahren der Rekonstruktion, ist es im Rahmen der Subtraktionsangiographie auch möglich, ein dreidimensionales Subtraktionsangiographiebild, also einen dreidimensionalen Subtraktionsangiographiedatensatz, zu erhalten, mithin ein dreidimensionales Abbild des Blutgefäßsystems des Patienten. Derartige dreidimensionale Bilddatensätze können beispielsweise im Rahmen der Navigation von medizinischen Instrumenten eingesetzt werden, beispielsweise dann, wenn der dreidimensionale Subtraktionsangiographiedatensatz mit dem Koordinatensystem einer Positionsbestimmungseinrichtung für das medizinische Instrument registriert ist.

Nichts desto trotz ist es auch bekannt, die Subtraktionsangiographie bei der fluoroskopischen Überwachung einzusetzen. Dabei wird ebenso von einem aufgenommenen Röntgenbild unter Kontrastmittel, also einem Füllungsbild, ein Maskenbild ohne Kontrastmittel abgezogen, um das letztendlich zur Überwachung zu verwendende Fluoroskopiebild zu erhalten.

Probleme treten bei der fluoroskopischen Bildüberwachung eines interventionellen Eingriffs immer dann auf, wenn sich verschiedene Blutgefäße in der zweidimensionalen Subtraktionsangiographie im Bereich des Zielgebiets, also der Pathologie, überlagern, was jedoch im Allgemeinen nicht zu vermeiden ist. Um das Zielgebiet richtig einschätzen zu können, wechselt die den Eingriff durchführende Person die Projektionsrichtung, was unter Umständen dazu führen kann, dass wichtige Teile der Pathologie nicht mehr ausreichend genau dargestellt werden können. Daher ist ein Kompromiss, was die Auswahl der Aufnahmegeometrie und somit der Projektionsrichtung angeht, nicht zu vermeiden. Problematisch an Überlagerungen ist beispielsweise, dass die Zuordnung von Blutgefäßen unklar ist. Handelt es sich bei dem Zielgebiet beispielsweise um ein zu behandelndes Aneurysma, ist bei einem ebenso in der zweidimensionalen Projektionsdarstellung zu sehenden Blutgefäß unklar, ob dieses Gefäß aus dem Aneurysma entspringt oder mit diesem überhaupt nicht in Kontakt steht. Das Dokument US2015/0213600 wird als nächstliegender Stand der Technik gegenüber dem Gegenstand des Anspruchs 1 angesehen.

Der Erfindung liegt daher die Aufgabe zugrunde, zur Bildüberwachung eines interventionellen, insbesondere minimalinvasiven Eingriffs unabhängig von der Wahl der Projektionsrichtung eine überlagerungsfreie Darstellung des Zielgebiets bereitzustellen.

Zur Lösung dieser Aufgabe ist erfindungsgemäß ein Verfahren zur Bildunterstützung eines Behandlers bei der interventionellen Behandlung eines Zielgebiets eines Patienten vorgesehen, welches folgende Schritte umfasst:
- Aufnahme eines dreidimensionalen Subtraktionsangiographiedatensatzes eines das Zielgebiet umfassenden Zielbereichs des Patienten,
- Auswahl einer das Zielgebiet umfassenden interessierenden Region in dem Subtraktionsangiographiedatensatz,
- Bestimmen einer eine Projektionsrichtung zur Aufnahme zweidimensionaler Fluoroskopiebilder zur Überwachung des Eingriffs realisierenden Aufnahmegeometrie mit einer Röntgeneinrichtung,
- Ermittlung von die interessierende Region in der Aufnahmegeometrie überlagernden Störblutgefäßen sowie von Anteile der Störblutgefäße in der Aufnahmegeometrie ohne Überlagerung durch andere Blutgefäße abbildenden Abbildungsbereichen durch Vorwärtsprojektion des Subtraktionsangiographiedatensatzes in der Projektionsrichtung,
- Bestimmung einer Verlaufsinformation der Störblutgefäße wenigstens im Bereich der interessierenden Region und in den Abbildungsbereichen und Eintragen der Verlaufsinformation in einen zweidimensionalen Vorwärtsprojektionsdatensatz,
- Aufnahme eines Fluoroskopiebildes in der Aufnahmegeometrie,
- Ermittlung von die Störblutgefäße in den Abbildungsbereichen zeigenden Bildpunkten in dem Fluoroskopiebild anhand der Verlaufsinformation und Ermittlung einer Darstellungsintensitätsinformation aus den an den Bildpunkten im Fluoroskopiebild gemessenen Intensitäten,
- Subtraktion eines anhand der Verlaufsinformation in der interessierenden Region und der Darstellungsintensitätsinformation ermittelten Maskenbildes der Störblutgefäße von dem Fluoroskopiebild in der interessierenden Region, und
- Darstellung des derart modifizierten Fluoroskopiebildes.

Bei dem Fluoroskopiebild handelt es sich dabei besonders bevorzugt um ein Überwachungsbild der zweidimensionalen Subtraktionsangiographie, um eine optimale Vergleichbarkeit zwischen dem dreidimensionalen Subtraktionsangiographiedatensatz und dem zweidimensionalen Fluoroskopiebild zu erreichen; das bedeutet, die Aufnahme des zweidimensionalen Fluoroskopiebildes umfasst die Messung eines Füllungsbildes in der Aufnahmegeometrie und die Subtraktion eines ebenso in der Aufnahmegeometrie aufgenommenen Maskenbildes. Der interventionelle Eingriff kann insbesondere ein minimalinvasiver Eingriff im Blutgefäßsystem des Patienten, insbesondere im Gehirn und/oder im Herzbereich, sein.

Die der Erfindung zugrundeliegende Idee ist es, die durch den dreidimensionalen Subtraktionsangiographiedatensatz gegebenen Hintergrundinformationen zu verwenden, um für die letztlich gewählte Aufnahmegeometrie in der Projektion überlagert dargestellte Störblutgefäße zu identifizieren und die Hintergrundinformationen des dreidimensionalen Subtraktionsangiographiedatensatzes weiterhin zu nutzen, um eine Abschätzung des Schwächungsbeitrags dieser Störblutgefäße in der interessierenden Region zu ermöglichen, die zur Korrektur in der interessierenden Region herangezogen werden kann. Denn sind die Projektionsrichtungen und die Positionen des Röntgenstrahlers und des Röntgendetektors bekannt, lassen sich leicht Abbildungsbereiche identifizieren, in denen das Störblutgefäß ohne Überlagerung durch weitere Strukturen, also Blutgefäße, erscheint, wenn ein Bild in der Aufnahmegeometrie aufgenommen wird. Findet man diese Abbildungsbereiche im Fluoroskopiebild wieder, kann dort davon ausgegangen werden, dass die gemessene Intensität, also der Bildwert, im Wesentlichen allein durch die Schwächung durch das Störblutgefäß entstand. Hieraus lassen sich Rückschlüsse über die Schwächung des Störblutgefäßes in der interessierenden Region ziehen, so dass für die interessierende Region ein Maskenbild geschaffen werden kann, das nur die Störblutgefäße und ihre Schwächungsanteile an der gemessenen Intensität enthält. Wird dieses Maskenbild vom Fluoroskopiebild subtrahiert, werden mithin auch die Störblutgefäße aufgrund der Entfernung ihrer Schwächungsanteile entfernt und es entsteht ein modifiziertes Fluoroskopiebild, in dem das Zielgebiet wie durch ein Fenster nichtüberlagert und deutlich zu erkennen ist.

Unter Verwendung des erfindungsgemäßen Verfahrens ist es also möglich, einem Betrachter in jeder technisch verfügbaren Aufnahmegeometrie eine uneingeschränkte Sicht auf den Behandlungsort, also das Zielgebiet, während der Überwachung im zweidimensionalen (DSA-)Fluoroskopiebild zu gewährleisten. Dies kann zur sicheren und schnelleren Behandlung führen. Besonders vorteilhaft lässt sich das Verfahren bei neurovaskulären Behandlungen einsetzen, beispielsweise Aneurysmen, AVM und dergleichen. Als insbesondere minimalinvasive Eingriffe können das Setzen von Stents, das Beseitigen von Okklusionen und dergleichen genannt werden.

Dabei sei an dieser Stelle noch angemerkt, dass vorgesehen sein kann, dass die Abbildungsbereiche insbesondere außerhalb der interessierenden Region gewählt werden können, wobei abhängig von deren Größe, die sich ja an dem (im dreidimensionalen Subtraktionsangiographiedatensatz mithin bekannten) Zielgebiet orientiert, auch Abbildungsbereiche innerhalb der interessierenden Region (ROI) zugelassen werden können. Insbesondere ist die interessierende Region jedoch meist eher eng um das Zielgebiet, beispielsweise einer Pathologie, herum zu setzen, was sowohl manuell als auch wenigstens teilweise automatisch erfolgen kann. Abgesehen vom Setzen der interessierenden Region, die einen Benutzereingriff beinhalten kann, werden alle Schritte des erfindungsgemäßen Verfahrens automatisch durchgeführt, beispielsweise durch eine Steuereinrichtung der Röntgeneinrichtung.

Zunächst erfolgt also die Aufnahme des dreidimensionalen Subtraktionsangiographiedatensatzes, wie dies im Stand der Technik grundsätzlich bekannt ist. Je nach den Eigenschaften der Röntgeneinrichtung, die hier verwendet wird, kann bereits eine einzige Kontrastmittelgabe ausreichend sein, um alle Füllungsbilder aus den unterschiedlichen Projektionsrichtungen aufzunehmen. Die Rekonstruktion dreidimensionaler Bilder aus den zweidimensionalen Projektionsbildern kann dabei unter Verwendung grundsätzlich bekannter Verfahren erfolgen, beispielsweise der gefilterten Rückprojektion und/oder der iterativen Rekonstruktion.

Besonders vorteilhaft ist in diesem Zusammenhang, wenn die Aufnahme des dreidimensionalen Subtraktionsangiographiedatensatzes mit derselben Röntgeneinrichtung wie das Fluoroskopiebild bei zwischenzeitlich unbewegtem Patienten erfolgt. Der Subtraktionsangiographiedatensatz wird bevorzugt vor oder zu Beginn des interventionellen Eingriffs aufgenommen, also dann, wenn der Patient bereits auf einem entsprechenden Patiententisch positioniert ist. Wird eine Röntgeneinrichtung mit einstellbaren Aufnahmegeometrien, insbesondere eine Röntgeneinrichtung mit einem C-Bogen, eingesetzt, lässt sich diese Röntgeneinrichtung auch zur Aufnahme von den dem dreidimensionalen Subtraktionsangiographiedatensatz zugrundeliegenden Projektionsbildern einsetzen, indem beispielsweise der C-Bogen um den Patienten herum verschwenkt wird und die Projektionsbilder aus unterschiedlichen Projektionsrichtungen aufgenommen werden. Wird dieselbe Röntgeneinrichtung nun auch zur Aufnahme der Fluoroskopiebilder verwendet und ist der Patient im Wesentlichen unbewegt, liegt bereits eine Registrierung vor, das bedeutet, die Aufnahmegeometrie und die Lage des Fluoroskopiebildes relativ zu dem dreidimensionalen Subtraktionsangiographiedatensatz sind bekannt, so dass ohne weitere Probleme die Vorwärtsprojektion vorgenommen werden und Verlaufsinformationen von dem Vorwärtsprojektionsdatensatz auf das Fluoroskopiebild übertragen werden können, wobei für den letztgenannten Schritt, wie im Folgenden noch näher dargelegt werden wird, eine Feinregistrierung eine zweckmäßige Erweiterung darstellen kann. In den Steuereinrichtungen moderner C-Bogen-Röntgeneinrichtungen sind meist ohnehin die Position des Röntgenstrahlers und des Röntgendetektors sowie ihre Ausrichtung bekannt, so dass ein Behandler eine gewünschte Aufnahmegeometrie einstellen kann, die dann auch unmittelbar bekannt ist und zur Verfügung steht, um die Vorabberechnungen zur überlagerungsfreien Darstellung des Zielgebiets vorzunehmen.

Es sei jedoch darauf hingewiesen, dass es grundsätzlich selbstverständlich auch denkbar ist, ein Koordinatensystem der Röntgeneinrichtung zur Aufnahme der Fluoroskopiebilder mit dem Subtraktionsangiographiedatensatz zu registrieren, wie dies im Stand der Technik grundsätzlich bekannt ist. Dies ist jedoch weniger bevorzugt als die Verwendung ein- und derselben Röntgeneinrichtung zur Aufnahme des Subtraktionsangiographiedatensatzes und des Fluoroskopiebildes bei unbewegtem Patienten.

Nichtsdestotrotz treten häufig Fälle auf, in denen der Patient, zumindest intern, nicht völlig unbewegt bleiben kann, sei es aufgrund natürlicher, periodischer Bewegung im Patienten oder aber aufgrund des Eingriffs selbst, beispielsweise hervorgerufen durch ein medizinisches Instrument. In solchen Fällen sieht eine zweckmäßige Weiterbildung der vorliegenden Erfindung vor, dass vor der Ermittlung der die Störblutgefäße in den Abbildungsbereichen zeigenden Bildpunkte eine Feinregistrierung des Vorwärtsprojektionsdatensatzes und des Fluoroskopiebildes aufgrund wenigstens des durch die Verlaufsinformation beschriebenen Verlaufs der Störblutgefäße in der interessierenden Region erfolgt. Nachdem mithin zumindest teilweise bekannt ist, wie die Störblutgefäße verlaufen, kann versucht werden, diese auch im Fluoroskopiebild aufzufinden und zur Deckung zu bringen, insbesondere, nachdem ja bereits eine hervorragende Ausgangsposition durch die grundlegende Registrierung gegeben ist. Beliebige 2D-2D-Registrierungsverfahren können hier eingesetzt werden, um eine möglichst exakte Überlagerung sicherzustellen, wobei durchaus auch eine elastische Registrierung denkbar ist, wenn beispielsweise eine Verformung von Blutgefäßen durch medizinische Instrumente zu befürchten ist oder dergleichen. Durch eine derartige Verfeinerung der Registrierung, also Feinregistrierung, lassen sich Effekte kleinerer Bewegungen und dergleichen kompensieren, so dass die Qualität des letztlich entstehenden, das Zielgebiet überlagerungsfrei zeigenden Fluporoskopiebildes nochmals deutlich erhöht werden kann.

Als Verlaufsinformation kann mit besonderem Vorteil eine Mittellinie und/oder eine Ausdehnung der Störblutgefäße ermittelt werden. Dabei sind bereits Bildauswertungsverfahren zur Analyse von Blutgefäßen bekannt, die insbesondere eine Mittellinie ("Centerline") ausgeben, in die auch jeweils an den entsprechenden Punkten der Mittellinie eine Ausdehnung des Blutgefäßes, beispielsweise eines Radius oder Durchmessers, an dieser Stelle integriert sein kann. Nachdem in dreidimensionalen Subtraktionsangiographiedatensätzen meist eine hohe Qualität durch Verbindung der Informationen vieler einzelner Projektionsbilder vorliegt, lässt sich der Verlauf, insbesondere beschrieben durch Mittellinie und Ausdehnung, dort äußerst genau ermitteln. Wird die Ausdehnung zudem in die Datenstruktur der Mittellinie integriert, ist eine äußerst kompakte Datenstruktur gegeben, die sich im weiteren leicht handhaben lässt.

Grundsätzlich, aber weniger bevorzugt, ist es alternativ auch denkbar, eine bei der Erstellung des Maskenbildes zu berücksichtigende Ausdehnung der Störblutgefäße durch Segmentierung der Störblutgefäße in dem Fluoroskopiebild wenigstens in der interessierenden Region, insbesondere im Rahmen der Feinregistrierung, zu erreichen. Solche Segmentierungen erweisen sich jedoch häufig aufgrund der vorliegenden Überlagerungen als etwas komplexer, so dass bevorzugt die hervorragende Information in dem dreidimensionalen Subtraktionsangiographiedatensatz genutzt wird.

Es sei an dieser Stelle noch angemerkt, dass es bevorzugt ist, die Verlaufsinformation für die Störblutgefäße zumindest soweit zu bestimmen, dass in ihr der komplette Verlauf der Störblutgefäße zumindest auch zwischen Abbildungsbereichen und/oder zwischen einem Abbildungsbereich und der interessierenden Region gegeben ist. Gerade dann, wenn nicht von konstanten Schwächungen über den Verlauf des Störblutgefäßes ausgegangen wird, mithin variierende Intensitätswerte entlang der Mittellinie im Maskenbild angenommen werden sollen, ist es nützlich, den Verlauf ausgehend von Stützstellen möglichst vollständig zu kennen.

Ein Vorteil des erfindungsgemäßen Vorgehens ist es, dass jegliche technisch realisierbare Aufnahmegeometrie im Rahmen des erfindungsgemäßen Verfahrens behandelt werden kann. Dies ist besonders vorteilhaft, wenn während des interventionellen Eingriffs, insbesondere während der fluoroskopischen Bildüberwachung, die Aufnahmegeometrie verändert wird. Während für mehrere Fluoroskopiebilder in derselben Aufnahmegeometrie der Vorwärtsprojektionsdatensatz selbstverständlich weiter verwendet werden kann, kann in bevorzugter Ausgestaltung der vorliegenden Erfindung vorgesehen sein, dass bei einer Änderung der Aufnahmegeometrie während der fluoroskopischen Bildüberwachung eine Neubestimmung des Vorwärtsprojektionsdatensatzes unter Verwendung der neuen Aufnahmegeometrie erfolgt und dieser Vorwärtsprojektionsdatensatz zur Korrektur folgend aufgenommener Fluoroskopiebilder der neuen Aufnahmegeometrie verwendet wird. Nachdem der dreidimensionale Subtraktionsangiographiedatensatz ja weiterhin vorliegt, lassen sich für neu eingestellte Aufnahmegeometrien mithin äußerst schnell entsprechende Störblutgefäße, diesen zugeordnete überlagerungsfreie Abbildungsbereiche und die Verlaufsinformationen bestimmen, so dass auch ein Wechsel der Aufnahmegeometrie während der Bildüberwachung vorteilhaft unterstützt wird.

In einer besonders vorteilhaften Ausgestaltung der vorliegenden Erfindung kann vorgesehen sein, dass eine Intensität der Darstellung der Störblutgefäße in dem Maskenbild durch Interpolation oder Extrapolation zwischen oder von an den Bildpunkten in den Abbildungsbereichen gemessenen Intensitäten ermittelt wird. Insbesondere also dann, wenn nicht davon ausgegangen werden kann, dass die Intensität, die durch die Schwächung des Störblutgefäßes allein entlang dessen Mittellinie entsteht, konstant bleibt, ist es zweckmäßig, die Intensität zwischen durch die Abbildungsbereiche gegebenen Stützstellen zu interpolieren bzw. ausgehend von den Abbildungsbereichen hin zur interessierenden Region zu extrapolieren, wozu zweckmäßig der gesamte Verlauf des Störblutgefäßes hin zur interessierenden Region bekannt ist. Solche Intensitätsschwankungen/Schwächungsschwankungen können von der Verteilung des Kontrastmittels in den Störblutgefäßen oder aber auch von Variationen in der Störblutgefäßstruktur herrühren. Durch die hier beschriebene Vorgehensweise ist es möglich, resultierende Effekte ebenso zu berücksichtigen.

Dabei wird es besonders bevorzugt, wenn durch die Interpolation oder Extrapolation ein Intensitätsverlauf entlang der als Teil der Verlaufsinformation ermittelten Mittellinie der Störblutgefäße bestimmt wird. Die Interpolation/Extrapolation kann mithin im Wesentlichen eindimensional entlang der Mittellinie erfolgen, was die Berechnungen deutlich vereinfacht und die Echtzeitfähigkeiten des erfindungsgemäßen Verfahrens unterstützt. Nachdem die allgemeine Form von Blutgefäßen bekannt ist, lässt sich der Verlauf der Schwächung senkrecht zu den Mittellinien leicht abschätzen, wozu insbesondere vorgesehen sein kann, dass eine gemäß der Ausdehnung parametrierbare Funktion zur Beschreibung des Abfalls der Schwächung zum Rand der Störblutgefäße hin im Maskenbild verwendet wird. Entsprechende Modelle von Blutgefäßen sind im Stand der Technik bereits bekannt und können hier eingesetzt werden, um eine entsprechende, geeignete Funktion auffinden zu können.

Gerade im Kontext einer Interpolation bzw. Extrapolation ist es besonders zweckmäßig, wenn als Abbildungsbereiche in der Projektionsrichtung überlagerungsfreie Abschnitte der Störblutgefäße möglichst nah an der interessierenden Region gewählt werden. Auf diese Weise wird der Abstand der bei der Interpolation/Extrapolation gegebenen Stützstellen von der interessierenden Region bzw. dem Zielgebiet möglichst gering gehalten.

Neben dem Verfahren betrifft die vorliegende Erfindung auch eine Röntgeneinrichtung, aufweisend eine zur Durchführung des erfindungsgemäßen Verfahrens ausgebildete Steuereinrichtung. Sämtliche Ausführungen bezüglich des erfindungsgemäßen Verfahrens lassen sich analog auf die erfindungsgemäße Röntgeneinrichtung übertragen, so dass auch mit dieser die bereits genannten Vorteile erhalten werden können. Insbesondere kann es sich um eine Röntgeneinrichtung mit einem C-Bogen, an dem sich gegenüberliegend ein Röntgenstrahler und ein Röntgendetektor befestigt sind, handeln. Derartige C-Bogen-Röntgeneinrichtungen werden besonders häufig in Angiographielaboren zur interventionellen Behandlung eingesetzt, da aufgrund des C-Bogens eine hohe Flexibilität besteht.

Schließlich betrifft die Erfindung auch ein Computerprogramm, das die Schritte des erfindungsgemäßen Verfahrens durchführt, wenn es auf einer Recheneinrichtung, insbesondere der genannten Steuereinrichtung der Röntgeneinrichtung, ausgeführt wird. Auch für das Computerprogramm gelten die Ausführungen zum Verfahren und zur Röntgeneinrichtung fort. Dabei sei an dieser Stelle noch darauf hingewiesen, dass bezüglich der Aufnahme des dreidimensionalen Subtraktionsangiographiedatensatzes und des Fluoroskopiebildes die Durchführung der Schritte selbstverständlich als eine Ansteuerung der entsprechenden Komponenten der Röntgeneinrichtung zu verstehen ist. Das Computerprogramm kann auf einem nicht transienten, elektronisch lesbaren Datenträger, insbesondere einer CD-ROM gespeichert sein.

Die das erfindungsgemäße Verfahren ausführende Steuereinrichtung als Recheneinrichtung kann neben einer Steuereinheit zur Ansteuerung von Komponenten der Röntgeneinrichtung zur Aufnahme von Röntgendaten auch eine Auswahleinheit zur Auswahl der interessierenden Region, eine Aufnahmegeometrieermittlungseinheit zur Ermittlung der aktuell eingestellten Aufnahmegeometrie, eine Vorwärtsprojektionsdatensatzermittlungseinheit zur Ermittlung des Vorwärtsprojektionsdatensatzes, eine Maskenbildermittlungseinheit zur Ermittlung des Maskenbildes und eine Darstellungseinheit zur Ausgabe des Fluoroskopiebildes an einer Darstellungseinrichtung der Röntgeneinrichtung aufweisen.

Weitere Vorteile und Einzelheiten der Erfindung ergeben sich aus den im Folgenden beschriebenen Ausführungsbeispielen sowie anhand der Zeichnungen. Dabei zeigen:
- Fig. 1: einen Ablaufplan eines Ausführungsbeispiels des erfindungsgemäßen Verfahrens,
- Fig. 2: ein noch nicht modifiziertes Fluoroskopiebild,
- Fig. 3: ein modifiziertes Fluoroskopiebild, und
- Fig. 4: eine erfindungsgemäße Röntgeneinrichtung.

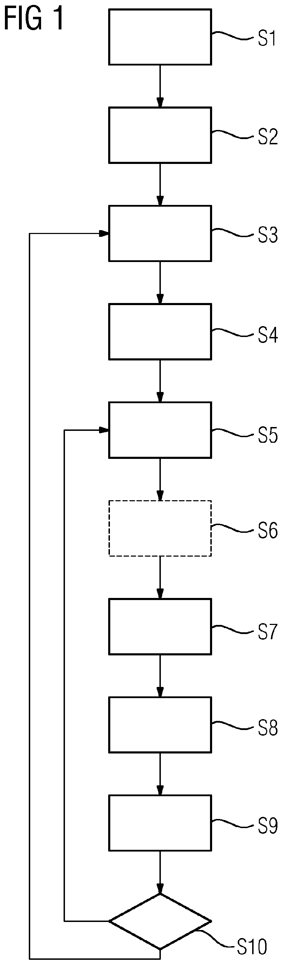

Fig. 1 zeigt einen Ablaufplan eines Ausführungsbeispiels des erfindungsgemäßen Verfahrens. Dieses dient zur Bildunterstützung bei der Überwachung eines interventionellen Eingriffs. Vorliegend kann beispielhaft ein minimalinvasiver, neurovaskulärer Eingriff betrachtet werden.

In einem Schritt S1 wird vor Beginn des Eingriffs oder aber zu Beginn desselben ein dreidimensionaler Subtraktionsangiographiedatensatz des das Zielgebiet des Eingriffs enthaltenden Gefäßsystems des zu behandelnden Patienten aufgenommen. Hierzu werden mithin Masken-Projektionsbilder und Füllungs-Projektionsbilder aus unterschiedlichen Projektionsrichtungen aufgenommen, woraus dann auf eine der bekannten Arten, umfassend eine Rekonstruktion, ein dreidimensionaler Subtraktionsangiographiedatensatz ermittelt werden kann.

In einem Schritt S2 wird automatisch und/oder manuell eine interessierende Region innerhalb des Subtraktionsangiographiedatensatzes festgelegt, die das Zielgebiet, vorzugsweise eng, umschließt. Beispielsweise kann die Zielregion von einem Benutzer mittels einer entsprechenden Benutzerschnittstelle markiert werden, woraufhin automatisch eine interessierende Region (ROI) in einem gewissen Abstand um das Zielgebiet gelegt wird.

In einem Schritt S3 wird eine Aufnahmegeometrie, umfassend eine Projektionsrichtung, bestimmt, die zur Aufnahme von Fluoroskopiebildern zur Bildüberwachung des Eingriffs während des Eingriffs genutzt werden soll, wobei die Fluoroskopiebilder mit derselben Röntgeneinrichtung wie der dreidimensionale Subtraktionsangiographiedatensatz aufgenommen werden sollen. Hat der Behandler eine von ihm gewünschte Aufnahmegeometrie eingestellt, ist diese in der Steuereinrichtung der Röntgeneinrichtung, die das hier beschriebene Verfahren durchführt, bekannt; die entsprechenden einstellbaren Komponenten liefern mithin entsprechendes Feedback an die Steuereinrichtung.

In einem Schritt S4 wird ein zweidimensionaler Vorwärtsprojektionsdatensatz ermittelt, der sich auf die aktuell gewählte Aufnahmegeometrie bezieht. Nachdem für die Aufnahme der Fluoroskopiebilder und die Aufnahme des Subtraktionsangiographiedatensatzes dieselbe Röntgeneinrichtung verwendet wird, zudem auch der Patient im Wesentlichen unbewegt bleibt, ist die Beziehung der Aufnahmegeometrie zu dem Subtraktionsangiographiedatensatz bekannt, so dass im Sinne einer Vorwärtsprojektion eine Aufnahme simuliert werden kann. Insbesondere wird an dieser Stelle zunächst ermittelt, welche Blutgefäße in Fluoroskopiebildern der Aufnahmegeometrie das Zielgebiet störend überlagern werden, welche im Folgenden als Störblutgefäße bezeichnet werden. Für Störblutgefäße gilt also, dass Strahlverläufe durch das Zielgebiet auch das Störblutgefäß durchqueren. Statt allein dem Zielgebiet kann auch die gesamte interessierende Region hier betrachtet werden, falls dies die Berechnungen vereinfacht. Sind die Störblutgefäße erst identifiziert, lässt sich anhand der bekannten Aufnahmegeometrie und der Vorwärtsprojektion auch ermitteln, wo die Störblutgefäße außerhalb der interessierenden Region ohne Überlagerungen, also ausschließlich, abgebildet werden. Solche bezüglich der Störblutgefäße überlagerungsfreien Bereiche werden als Abbildungsbereiche bezeichnet und gespeichert. Vorliegend werden als Abbildungsbereiche solche überlagerungsfrei die Störblutgefäße abbildenden Regionen gewählt, die sich möglichst nah am Zielgebiet bzw. der interessierenden Region befinden. Schließlich wird noch der zweidimensionale Verlauf der Störblutgefäße in der Vorwärtsprojektion wenigstens in den Abbildungsbereichen und der interessierenden Region ermittelt und als Verlaufsinformation neben der Lage der Abbildungsbereiche und der interessierenden Region in dem Vorwärtsprojektionsdatensatz gespeichert. Vorliegend werden die Mittellinie und die Ausdehnung der Störblutgefäße ermittelt, wobei die Ausdehnung in eine Datenstruktur der Mittellinie hineinkodiert werden kann, um ein möglichst kompaktes Speichern der Daten zu erlauben.

In einem Schritt S5 erfolgt dann, häufig ausgelöst durch die Betätigung eines Fußtasters durch eine den Eingriff durchführende bzw. an dem Eingriff beteiligte Person, die Aufnahme eines zweidimensionalen Fluoroskopiebildes, wobei das zweidimensionale Fluoroskopiebild ein zweidimensionales Subtraktionsangiographiebild ist. Das bedeutet, durch die Röntgeneinrichtung wird ein Füllungsbild gemessen, wovon ein Maskenbild abgezogen wird, um das Fluoroskopiebild zu erhalten.

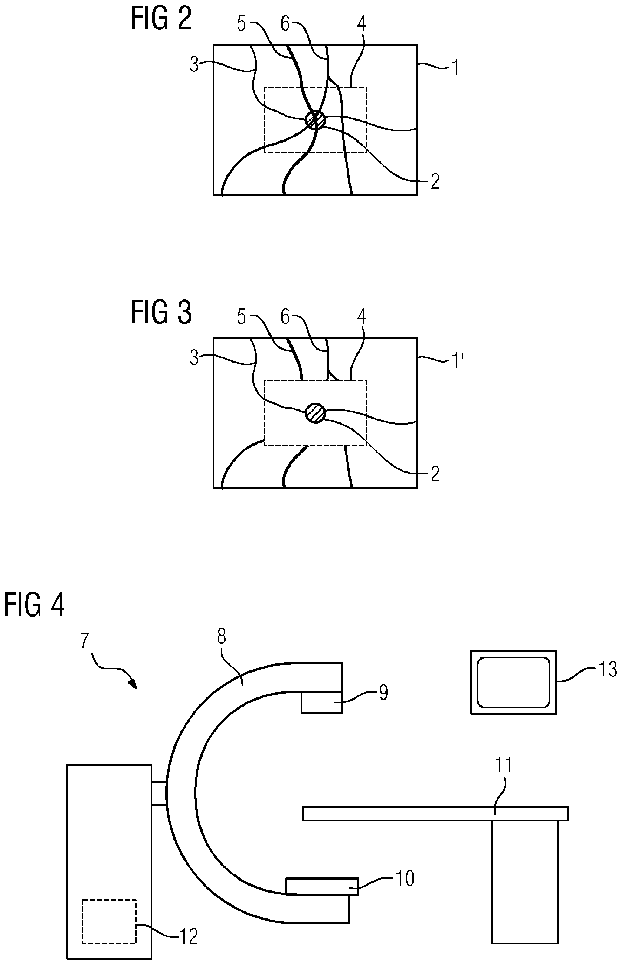

Schematisch und rein beispielhaft ist ein solches Fluoroskopiebild 1 in Fig. 2 dargestellt. Das Zielgebiet 2, beispielsweise ein Aneurysma, liegt entlang eines Blutgefäßes 3. Die angedeutete interessierende Region 4 umschließt das Zielgebiet 2 ersichtlich recht eng. Die Darstellung des Zielgebiets 2 wird jedoch überlagert von Störblutgefäßen 5, 6, die die Einschätzung des Zielgebiets erschweren. Die nun folgenden Schritte nutzen den zweidimensionalen Vorwärtsprojektionsdatensatz des Schrittes S4, um in der interessierenden Region 4 die Darstellung der Störblutgefäße 5, 6, die mit dem Zielgebiet 2 nicht in direktem Kontakt stehen, zu entfernen.

In einem optionalen Schritt S6 findet zunächst eine Feinregistrierung, also eine Verfeinerung der bereits gegebenen Registrierung statt, das bedeutet, die Verlaufsinformationen werden genutzt, um das Fluoroskopiebild 1 und den zweidimensionalen Vorwärtsprojektionsdatensatz möglichst genau zur Deckung zu bringen. Auf diese Weise können kleinere Bewegungen des Patienten und/oder Veränderungen aufgrund eines eingesetzten medizinischen Instruments ausgeglichen werden.

Der Schritt S7 dient sodann der Ermittlung eines Maskenbildes für die interessierende Region. Der Verlauf der Störblutgefäße 5, 6 innerhalb der interessierenden Region 4 ist ja bereits durch die Verlaufsinformationen bekannt. Um für ein Maskenbild eine entsprechende Darstellung der Störblutgefäße 5, 6 in der interessierenden Region 4 mit Intensitätswerten zu befüllen, werden die Abbildungsbereiche genutzt. Durch die Abbildungsbereiche und die vorhandene Registrierung sind schließlich auch die Bildpunkte im Fluoroskopiebild 1 bekannt, an denen das jeweilige Störblutgefäß 5, 6 überlagerungsfrei dargestellt ist; das bedeutet, die dort im Fluoroskopiebild enthaltenen Intensitätswerte rühren allein von der Schwächungswirkung des entsprechenden Störblutgefäßes 5, 6 her. Die entsprechenden Intensitätswerte in den Abbildungsbereichen, betrachtet entlang der Mittellinie, stellen nun Stützstellen dar, um mithilfe der Verlaufsinformationen Intensitätswerte für eine gedachte nicht überlagerte Darstellung der Störblutgefäße 5, 6 auch innerhalb der interessierenden Region 4 zu ermitteln, wobei beispielsweise eine lineare Interpolation zwischen der interessierenden Region gegenüberliegend benachbarten Abbildungsbereichen stattfinden kann, aber auch komplexere Ansätze verwendet werden können. Sollte, was weniger bevorzugt ist, ein Abbildungsbereich nur zu einer Seite der interessierenden Region 4 vorliegen, kann auch eine Extrapolation erfolgen.

Nach der Interpolation ist also ein Intensitätsverlauf entlang der Mittellinie der Störgefäße 5, 6 insbesondere auch innerhalb der interessierenden Region 4 bekannt, welcher nun genutzt wird, um das Maskenbild dort, wo die Störblutgefäße 5, 6 in der interessierenden Region 4 vorliegen, mit Intensitätswerten zu befüllen, die deren Schwächungsanteil anzeigen. Dabei werden die Schwächungsanteile bei sich überlagernden Störblutgefäßen 5, 6 selbstverständlich entsprechend kumuliert. Um den Intensitätsverlauf auch zum Rand der Störblutgefäße 5, 6 hin möglichst korrekt wiedergeben zu können, fällt der entsprechende Intensitätswert im Maskenbild zum Rand hin gemäß einer Funktion, die auf einem Blutgefäßmodell basiert, ab. Die Funktion ist mit den entsprechenden Ausdehnungen parametriert.

Das entstehende Maskenbild enthält also gerade und ausschließlich die Beiträge der Störblutgefäße 5, 6 innerhalb der interessierenden Region 4.

In einem Schritt S8 wird das Maskenbild in der interessierenden Region 4 von dem Fluoroskopiebild 1 abgezogen, so dass ein korrigiertes Fluoroskopiebild 1' entsteht, wie durch Fig. 3 angedeutet ist. Wie dort ersichtlich, sind die Beiträge der Störblutgefäße 5, 6 innerhalb der interessierenden Region 4 entfernt worden, so dass ein unverstellter Blick auf das Zielgebiet 2 gewährleistet ist. Auch gemäß dem Verfahren, vgl. Fig. 1, wird das Fluoroskopiebild in einem Schritt S9 an einer Anzeigevorrichtung der Röntgeneinrichtung dargestellt.

In einem Schritt S10 wird überprüft, ob sich die Aufnahmegeometrie ändert. Ist dies nicht der Fall, wird bei der Aufnahme des nächsten Fluoroskopiebildes wieder wie gewohnt mit Schritt S5 fortgefahren. Der Vorwärtsprojektionsdatensatz bleibt dann mithin unverändert. Ändert sich jedoch die Aufnahmegeometrie, beispielsweise, weil das Zielgebiet 2 aus einem anderen Blickwinkel betrachtet werden soll, wird die neue Aufnahmegeometrie gemäß Schritt S3 bestimmt und es wird entsprechend für die Aufnahmegeometrie ein neuer Vorwärtsprojektionsdatensatz im Schritt S4 ermittelt, der dann entsprechend zur Modifikation des Fluoroskopiebildes 1 zur Entfernung der Anteile von Störblutgefäßen 5, 6 verwendet wird.

Fig. 4 zeigt schließlich eine Prinzipskizze einer erfindungsgemäßen Röntgeneinrichtung 7. Die Röntgeneinrichtung 7 weist einen C-Bogen 8 auf, an dem sich gegenüberliegend ein Röntgenstrahler 9 und ein Röntgendetektor 10 angeordnet sind. Dank der Bewegbarkeit des C-Bogens 8 können so verschiedene Aufnahmegeometrien bezüglich eines auf einem Patiententisch 11 platzierten Patienten (hier nicht dargestellt) eingestellt werden. Die Röntgeneinrichtung 7 weist ferner eine Steuereinrichtung 12 auf, die zur Durchführung des erfindungsgemäßen Verfahrens ausgebildet ist, sowie eine Anzeigevorrichtung 13, hier ein Monitor, zur Darstellung des modifizierten Fluoroskopiebildes 1'.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zur Bildunterstützung eines Behandlers bei der interventionellen Behandlung eines Zielgebiets (2) eines Patienten, umfassend folgende Schritte:
- Aufnahme eines dreidimensionalen Subtraktionsangiographiedatensatzes eines das Zielgebiet (2) umfassenden Zielbereichs des Patienten,
- Auswahl einer das Zielgebiet (2) umfassenden interessierenden Region (4) in dem Subtraktionsangiographiedatensatz,
- Bestimmen einer eine Projektionsrichtung zur Aufnahme zweidimensionaler Fluoroskopiebilder (1) zur Überwachung des Eingriffs realisierenden Aufnahmegeometrie mit einer Röntgeneinrichtung (7),
- Ermittlung von die interessierende Region (4) in der Aufnahmegeometrie überlagernden Störblutgefäßen (5, 6) sowie von Anteile der Störblutgefäße (5, 6) in der Aufnahmegeometrie ohne Überlagerung durch andere Blutgefäße abbildenden Abbildungsbereichen durch Vorwärtsprojektion des Subtraktionsangiographiedatensatzes in der Projektionsrichtung,
- Bestimmung einer Verlaufsinformation der Störblutgefäße (5, 6) wenigstens im Bereich der interessierenden Region (4) und in den Abbildungsbereichen und Eintragen der Verlaufsinformation in einen zweidimensionalen Vorwärtsprojektionsdatensatz,
- Aufnahme eines Fluoroskopiebildes (1) in der Aufnahmegeometrie,
- Ermittlung von die Störblutgefäße (5, 6) in den Abbildungsbereichen zeigenden Bildpunkten in dem Fluoroskopiebild (1) anhand der Verlaufsinformation und einer Darstellungsintensitätsinformation aus den an den Bildpunkten im Fluoroskopiebild (1) gemessenen Intensitäten,
- Subtraktion eines anhand der Verlaufsinformation in der interessierenden Region (4) und der Darstellungsintensitätsinformation ermittelten Maskenbildes der Störblutgefäße (5, 6) von dem Fluoroskopiebild (1) in der interessierenden Region (4), und
- Darstellung des derart modifizierten Fluoroskopiebildes (1').

2. Verfahren nach Anspruch 1, **dadurch gekennzeichnet, dass** die Aufnahme des dreidimensionalen Subtraktionsangiographiedatensatzes mit derselben Röntgeneinrichtung (7) wie das Fluoroskopiebild (1) bei zwischenzeitlich unbewegtem Patienten erfolgt oder ein Koordinatensystem der Röntgeneinrichtung (7) zur Aufnahme der Fluoroskopiebilder (1) mit dem Subtraktionsangiographiedatensatz registriert ist oder wird.

3. Verfahren nach Anspruch 1 oder 2, **dadurch gekennzeichnet, dass** vor der Ermittlung der die Störblutgefäße (5, 6) in den Abbildungsbereichen zeigenden Bildpunkte eine Feinregistrierung des Vorwärtsprojektionsdatensatzes und des Fluoroskopiebilds (1) aufgrund wenigstens des durch die Verlaufsinformationen beschriebenen Verlaufs der Störblutgefäße (5, 6) in der interessierenden Region (4) erfolgt.

4. Verfahren nach einem der vorangehenden Ansprüche, **dadurch gekennzeichnet, dass** die Verlaufsinformation eine Mittellinie und/oder eine Ausdehnung der Störblutgefäße (5, 6) umfasst und/oder eine bei der Erstellung des Maskenbildes zu berücksichtigende Ausdehnung der Störblutgefäße (5, 6) durch Segmentierung der Störblutgefäße (5, 6) in dem Fluoroskopiebild (1) wenigstens in der interessierenden Region (4), insbesondere im Rahmen der Feinregistrierung, ermittelt wird.

5. Verfahren nach einem der vorangehenden Ansprüche, **dadurch gekennzeichnet, dass** bei einer Änderung der Aufnahmegeometrie während der fluoroskopischen Bildüberwachung eine Neubestimmung des Vorwärtsprojektionsdatensatzes unter Verwendung der neuen Aufnahmegeometrie erfolgt und dieser Vorwärtsprojektionsdatensatz zur Korrektur folgend aufgenommener Fluoroskopiebilder (1) der neuen Aufnahmegeometrie verwendet wird.

6. Verfahren nach einem der vorangehenden Ansprüche, **dadurch gekennzeichnet, dass** eine Intensität der Darstellung der Störblutgefäße (5, 6) in dem Maskenbild durch Interpolation oder Extrapolation zwischen oder von an den Bildpunkten in den Abbildungsbereichen gemessenen Intensitäten ermittelt wird.

7. Verfahren nach Anspruch 6, **dadurch gekennzeichnet, dass** durch die Interpolation oder Extrapolation ein Intensitätsverlauf entlang der als Teil der Verlaufsinformation ermittelten Mittellinie der Störblutgefäße (5, 6) bestimmt wird.

8. Verfahren nach Anspruch 7, **dadurch gekennzeichnet, dass** eine gemäß der Ausdehnung parametrierbare Funktion zur Beschreibung des Abfalls der durch die Intensität beschriebenen Schwächung zum Rand der Störblutgefäße (5, 6) hin im Maskenbild verwendet wird.

9. Verfahren nach einem der Ansprüche 6 bis 8, **dadurch gekennzeichnet, dass** als Abbildungsbereiche in der Projektionsrichtung überlagerungsfreie Abschnitte der Störblutgefäße (5, 6) möglichst nah an der interessierenden Region (4) gewählt werden.

10. Röntgeneinrichtung (7), aufweisend eine zur Durchführung eines Verfahrens nach einem der vorangehenden Ansprüche ausgebildete Steuereinrichtung (12).

11. Computerprogramm, das die Schritte eines Verfahrens nach einem der Ansprüche 1 bis 8 durchführt, wenn es auf einer Recheneinrichtung ausgeführt wird.

## Claims

1. Method for providing image support to a practitioner in the interventional treatment of a target area (2) in a patient, including the following steps:
- acquisition of a three-dimensional subtraction angiography image data set including a target region of the patient that includes the target area (2),
- selection of a region of interest (4) in the subtraction angiography data set, which region includes the target area (2),
- defining an imaging geometry that implements a projection direction for acquiring two-dimensional fluoroscopic images (1) for monitoring the intervention using an X-ray device (7),
- determination of the image-obscuring blood vessels (5, 6) that superimpose the region of interest (4) in the imaging geometry, and of imaging zones that show fractions of the image-obscuring blood vessels (5, 6) in the imaging geometry without superimposition by other blood vessels by forward projection of the subtraction angiography data set in the projection direction,
- defining path information relating to the image-obscuring blood vessels (5, 6) at least in the zone around the region of interest (4) and in the imaging zones, and input of the path information into a two-dimensional forward projection data set,
- acquisition of a fluoroscopic image (1) in the imaging geometry,
- determination of the pixels that show the image-obscuring blood vessels (5, 6) in the fluoroscopic image (1) by means of the information relating to the path, and of image intensity information from the intensities measured on the pixels in the fluoroscopic image (1),
- subtraction from the fluoroscopic image (1) in the region of interest (4), of a masked image of the image-obscuring blood vessels (5, 6) acquired by means of the path information in region of interest (4) and the image intensity information and
- display of the fluoroscopic image that has been modified in this way (1').

2. Method according to claim 1, **characterised in that** the acquisition of the three-dimensional image data set ensues with the same X-ray device (7) as the fluoroscopic image (1) in the patient who has meanwhile not been moved, or a coordinate system of the X-ray device (7) is or becomes registered with the subtraction angiography data set in order to acquire the fluoroscopic images (1).

3. Method according to claim 1 or 2, **characterised in that**, before the acquisition of the image-obscuring blood vessels (5, 6) in the pixels showing the imaging zones, a fine registration of the forward projection data set and of the fluoroscopic image (1) ensues on the basis of at least the path of the image-obscuring blood vessels (5, 6) in the region of interest (4) described by the information relating to the path.

4. Method according to one of the preceding claims, **characterised in that** the path information includes at least a centre line and/or an extent of the image-obscuring blood vessels (5, 6) and/or an extent of the image-obscuring blood vessels (5, 6) that is to be considered when generating the masked image in the fluoroscopic image (1) is determined at least in the region of interest (4), in particular in the context of the fine registration.

5. Method according to one of the preceding claims, **characterised in that**, where there is a change in the imaging geometry during the fluoroscopic image monitoring, a fresh definition of the forward projection data set ensues using the new imaging geometry and this forward projection data set is subsequently used to correct fluoroscopic images (1) of the new imaging geometry.

6. Method according to one of the preceding claims, **characterised in that** an intensity of the imaging of the image-obscuring blood vessels (5, 6) in the masked image is acquired by interpolation or extrapolation between or from intensities measured on the pixels in the imaging zones.

7. Method according to claim 6, **characterised in that**, through the interpolation or extrapolation, an intensity trajectory is defined along the centre line of the image-obscuring blood vessels (5, 6) that has been acquired as part of the path information.

8. Method according to claim 7, **characterised in that** a function that can be parameterised according to the extent is used in the masked image towards the edge of the image-obscuring blood vessels (5, 6) to describe the decrease in the attenuation described by the intensity.

9. Method according to one of claims 6 to 8, **characterised in that** superimposition-free segments of the image-obscuring blood vessels (5, 6) are selected as imaging zones in the projection direction as close as possible to the region of interest (4).

10. X-ray device (7), comprising a control device (12) that is designed to carry out a method according to one of the preceding claims.

11. Computer program that implements the steps in a method according to one of claims 1 to 8 when it is run on a computation device.

## Revendications

1. Procédé pour assister en image un intervenant lors du traitement avec intervention d'une zone cible d'un patient, comprenant les stades suivants :
- enregistrement d'un jeu de données d'angiographie soustractive en trois dimensions d'une partie cible, comprenant la zone cible, du patient,
- choix dans le jeu de données d'angiographie soustractive d'une région (4) intéressante comprenant la zone (2) cible,
- détermination par un équipement (7) à rayon X d'une géométrie d'enregistrement réalisant, pour le contrôle de l'intervention, un sens de projection pour l'enregistrement d'images (1) de fluoroscopie en deux dimensions,
- détermination, par projection vers l'avant du jeu de données d'angiographie soustractive dans le sens de projection, de vaisseaux (5, 6) sanguins perturbés se superposant à la région (4) intéressante dans la géométrie d'enregistrement ainsi que de parties de vaisseaux (5, 6) sanguins perturbés dans la géométrie d'enregistrement sans superposition par des parties de reproduction reproduisant d'autres vaisseaux sanguins par projection vers l'avant du jeu de données d'angiographie soustractive dans le sens de projection,
- détermination d'une information de tracé des vaisseaux (5, 6) sanguins perturbés au moins dans la partie de la région (4) intéressante et dans les parties de reproduction et insertion de l'information de tracé dans un jeu de données en deux dimensions de projection vers l'avant,
- enregistrement d'une image (1) de fluoroscopie dans la géométrie d'enregistrement,
- détermination des points image, montrant les vaisseaux (5, 6) sanguins perturbés dans les parties de reproduction, dans l'image (1) de fluoroscopie à l'aide de l'information de tracé et d'une information d'intensité de représentation à partir des intensités mesurées aux points d'image de l'image (1) de fluoroscopie,
- soustraction de l'image (1) de fluoroscopie dans la région (4) intéressante, d'une image de masque, déterminée à l'aide de l'information de tracé dans la région (4) intéressante et de l'information d'intensité de représentation, des vaisseaux (5, 6) sanguins perturbés et
- représentation de l'image (1') de fluoroscopie ainsi modifiée.

2. Procédé suivant la revendication 1, **caractérisé en ce que** l'enregistrement du jeu de données d'angiographie soustractive en trois dimensions s'effectue par le même équipement (7) à rayon X que l'image (1) de fluoroscopie, sans déplacement du patient entre temps, ou un système de coordonnées de l'équipement (1) à rayons X est enregistré pour l'enregistrement des images (1) de fluoroscopie avec le jeu de données d'angiographie soustractive.

3. Procédé suivant la revendication 1 ou 2, **caractérisé en ce qu'**avant la détermination des points image montrant les vaisseaux (5, 6) sanguins perturbés dans les parties de reproduction, il est effectué un enregistrement fin des jeux de données de projection vers l'avant et de l'image (1) de fluoroscopie, sur la base d'au moins le tracé, décrit par l'information de tracé, des vaisseaux (5, 6) sanguins perturbés dans la région (4) intéressante.

4. Procédé suivant l'une des revendications précédentes, **caractérisé en ce que** l'information de tracé comprend une ligne médiane et/ou une étendue des vaisseaux (5, 6) sanguins perturbés et/ou on détermine, notamment dans le cadre de l'enregistrement fin, une étendue, prise en compte lors de l'établissement de l'image de masque, des vaisseaux (5, 6) sanguins perturbés par segmentation des vaisseaux (5, 6) sanguins perturbés dans l'image (1) de fluoroscopie, au moins dans la région (4) intéressante.

5. Procédé suivant l'une des revendications précédentes, **caractérisé en ce que** s'il se produit une modification de la géométrie d'enregistrement avant le contrôle d'image fluoroscopique, on effectue une nouvelle détermination du jeu de données de projection vers l'avant en utilisant la nouvelle géométrie d'enregistrement et on utilise ce jeu de données de projection vers l'avant pour la correction d'images (1) de fluoroscopie enregistrées ensuite de la nouvelle géométrie d'enregistrement.

6. Procédé suivant l'une des revendications précédentes, **caractérisé en ce que** l'on détermine une intensité de la représentation des vaisseaux (5, 6) sanguins perturbés dans l'image de masque par interpolation ou par extrapolation entre ou à partir d'intensités mesurées aux points image dans les parties de reproduction.

7. Procédé suivant la revendication 6, **caractérisé en ce que** l'on définit, par l'interpolation ou l'extrapolation, un tracé d'intensité le long de la ligne médiane, déterminée comme partie de l'information de tracé, des vaisseaux (5, 6) sanguins perturbés.

8. Procédé suivant la revendication 7, **caractérisé en ce que** l'on utilise une fonction paramétrable suivant l'étendue pour décrire la décroissance de l'affaiblissement, décrit par l'intensité, vers le bord des vaisseaux (5, 6) sanguins perturbés dans l'image de masque.

9. Procédé suivant l'une des revendications 6 à 8, **caractérisé en ce que** l'on choisit, comme parties de reproduction, des segments sans superposition dans le sens de projection, des vaisseaux (5, 6) sanguins perturbés aussi proches que possible de la région (4) intéressante.

10. Equipement (7) à rayons X ayant un dispositif (12) de commande constitué pour effectuer un procédé suivant l'une des revendications précédentes.

11. Programme d'ordinateur, qui effectue les stades d'un procédé suivant l'une des revendications 1 à 8, lorsqu'il est réalisé sur un dispositif informatique.
